# FASCICULE DE BREVET EUROPEEN

(11) **EP 1 124 566 B1**
(45) Date de publication et mention de la délivrance du brevet: **12.02.2003**
(21) Numéro de dépôt: 99916994.9
(22) Date de dépôt: 05.05.1999
(51) Int. Cl.: A61K 31/765, A61P 35/00

(54) **LAXATIF OSMOTIQUE NON FERMENTE POUR TRAITER ET PREVENIR LES CANCERS COLORECTAUX**
OSMOTISCHES NICHT-FERMENTIERTES LAXANS ZUR BEHANDLUNG UND VORBEUGUNG VON KOLOREKTALEM KREBS
NON-FERMENTED OSMOTIC LAXATIVE FOR TREATING AND PREVENTING COLORECTAL CANCERS

(30) Priorité: 27.10.1998 FR 9813450; 16.03.1999 FR 9903240
(43) Date de publication de la demande: 22.08.2001
(73) Titulaire: INSTITUT NATIONAL DE LA RECHERCHE AGRONOMIQUE (INRA), 75338 Paris Cédex 07 (FR); Ecole Nationale Vétérinaire de Toulouse (ENVT), 31076 Toulouse Cedex 03 (FR)
(72) Inventeur: CORPET, Denis, E., F-31000 Toulouse (FR); TACHE, Sylviane, F-31490 Leguevin (FR); PARNAUD, Géraldine, F-66390 Baixas (FR)
(74) Mandataire: Breese, Pierre
(86) Numéro de dépôt international: FR9901065
(87) Numéro de publication internationale: WO00024407

(56) Documents cités:
- EP-A- 0 444 625
- CROWSON ET AL: "the use and efficacy of cytocidal agents in colorectal cancer" SURGICAL RES. COM., vol. 2, no. 2, 1987, pages 97-101, XP002111237
- HOSODA ET AL: "antitumor activity of doxorubicin encapsulated in P(EG)-coated liposomes" BIOL. PHARM. BUL., vol. 18, no. 9, 1995, pages 1234-1237, XP000533275

## Description

La présente invention concerne la prévention et le traitement des adénomes et des cancers du côlon et du rectum. Ces tumeurs sont, chez les non-fumeurs, la première cause de mortalité cancéreuse.

Les traitements proposés à ce jour sont basés sur l'exérèse chirurgicale des tumeurs, avec chimiothérapie adjuvante, utilisant par exemple du 5-Fluoro-Uracile. Ceux-ci présentent de nombreux inconvénients parmi lesquels on peut citer plus particulièrement un taux d'échec important (40% de survie à cinq ans), un coût élevé pour la société et des souffrances élevées pour le malade.

Il est également envisagé de prévenir les cancers digestifs par l'administration chronique d'aspirine, ou d'un AINS équivalent. Toutefois ce type de traitement entraîne encore des effets secondaires importants et reste relativement inefficace.

Le but de la présente invention est précisément de fournir de nouveaux moyens, facile d'emploi, pour prévenir et/ou guérir efficacement les adénomes et cancers du côlon et du rectum.

Ce but est atteint grâce à l'utilisation d'un laxatif osmotique non fermenté en tant qu'agent actif pour la préparation d'un médicament destiné au traitement et/ou à la prévention des cancers du côlon et/ou du rectum. De préférence les laxatifs osmotiques non fermentés entrant dans le cadre de la présente invention sont des polyols.

On entend au sens de la présente invention par laxatif, tout composé présentant des propriétés laxatives et/ou gélifiantes. Il s'agit de composés capables d'attirer et de retenir l'eau à l'intérieur du côlon du fait de leur propriété physico-chimique, et d'augmenter l'excrétion fécale à l'exclusion des fibres. En effet, il est connu que la consommation de fibres augmente la masse et l'hydratation des fèces. Mais les fibres sont fermentées par la microflore intestinale en acides gras volatils, lesquels participent à l'effet laxatif des fibres. L'un des acides gras volatils, le butyrate, serait impliqué dans l'effet protecteur des fibres sur les cancers colorectaux. Or, il a été observé que le butyrate normalise la croissance de cellules tumorales *in vitro,* et *in vivo* le butyrate augmenterait l'apoptose et donc le suicide des cellules tumorales. Trois grands types de fibres ont été étudiés pour leur effet de prévention du cancer du côlon :
- les fibres insolubles, comme le son de blé et la cellulose,
- les fibres solubles comme le son d'avoine, le psyllium ou l'hispagule (Alabaster, O., Tang, Z. C., Frost, A. and Shivapurkar, N. (1993) Potential synergism between wheat bran and psyllium - enhanced inhibition of colon cancer. Cancer Letters. 75: 53-58),
- les sucres indigestes comme l'inuline ou le lactulose (Challa, A., Rao, D. R., Chawan, C. B. and Shackelford, L. (1997) Bifidobacterium longum and lactulose suppress azoxymethane-induced colonic aberrant crypt foci in rats. Carcinogenesis. 18: 517-521 ; Reddy, B. S., Hamid, R. and Rao, C. V. (1997) Effect of dietary oligofructose and inulin on colonie preneoplastic aberrant crypt foci inhibition. Carcinogenesis. 18: 1371-1374 ; Roncucci, L., DiDonato, P., and PonzDeLeon, M. (1993) Antioxidant vitamins or lactulose for the prevention of the recurrence of colorectal adenomas. Dis Colon Rectum. 36: 227-234 ; Rowland, I. R., Rumney, C. J., Coutts, J. T. and Lievense, L. C. (1998) Effect of bifidobacterium longum and inulin on gut bacterial metabolism and carcinogen-induced aberrant crypt foci in rats. Carcinogenesis. 19: 281-285).

Ces trois types de fibres, auxquels on peut ajouter l'amidon résistant à la digestion, présentent des propriétés protectrices contre le cancer.

Les laxatifs osmotiques non fermentés comme le PEG, ne sont pas des fibres, car :
- ils ne sont pas de nature chimique polyosidique (enchaînement de sucres simples),
- ils ne sont pas fermentés par la microflore intestinale et ne donnent pas de butyrate,
- ils ne sont pas issus de parois végétales.

De façon remarquable, ces laxatifs osmotiques non fermentés comme le PEG, présentent un effet anti-cancer qui n'est pas lié au butyrate et qui est beaucoup plus puissant que celui des fibres, et leur propriété laxative sont vraisemblablement due à leur effet osmotique.

Parmi les laxatifs osmotiques non fermentés, l'invention concerne plus particulièrement l'utilisation de polyéthylène-glycol ou de polyéthylènepolypropylène glycol, un mélange ou un dérivé de ceux-ci.

Le polyéthylène-glycol (PEG) est un polymère bien connu, utilisé notamment comme adjuvant dans des compositions pharmaceutiques, répondant à la formule (I) suivante :

H-(O-CH₂-CH₂)ₙ-OH

dans laquelle n est de l'ordre de 200 pour les PEG de haut poids moléculaire, par exemple le PEG 8000.

Les PEG de haut poids moléculaire, c'est-à-dire supérieurs à environ 3000, sont connus pour leurs effets laxatifs. On considère que le PEG attire et retient l'eau dans l'intestin par un effet osmotique (Davis, G.R., SantaAna, C.A., et al., 1980, Gastroenterology, 79 : 35-39). Ainsi le PEG est utilisé chez l'homme dans diverses préparations soit en lavement préparatoire avant côlonoscopie (4 litres, 236 à 360 g de PEG), soit comme laxatif (3 à 40g/j). A titre d'exemples de telles préparations, on peut citer :
- Le Transipeg® qui est un laxatif iso-osmotique à base de macrogol (PEG de poids moléculaire 3350), se présentant sous la forme de sachet contenant 2,95 g de PEG et un excipient constitué de NaCl, Na₂SO₄ anhydre, Na₂CO₃, arôme pomme, aspartam. Le macrogol est un polymère sur lequel sont retenues les molécules d'eau par liaisons hydrogène. Il n'y aurait pas de résorption ni de bio-transformation du PEG qui reste confiné dans l'intestin (Vidal 1996, p.1625).
- Le Forlax® 10 g qui se présente sous la forme d'une poudre pour solution buvable constituée de mocrogol 4000 et d'un excipient fait de saccharine sodique, arôme orange-pamplemousse (Vidal 1996, p.649).
- Le Colyte® et le Golytely® qui est commercialisé aux USA et qui contient du PEG de poids moléculaire 3350. Le Colyte® a été proposé récemment pour favoriser la survie de souris CFTR dans un modèle de mucoviscidose (Clarke, L.L., Gawenis, L.R., Franklin, C.L., Harline M.C., 1996, Lab. Anim, Sci. 46 : 612-618).

L'efficacité de 20 g de PEG 4000, sans excipient ni électrolytes, pour lutter contre la constipation chronique a également été montrée dans un essai récent randomisé en double aveugle (Hudziak, H., Bronowicki, J.P., et al., 1996, Gastroenterol. Clin. Biol. 20 : 418-423).

De plus, un test effectué sur des cellules de cancer du colon HT29 n'a pas montré un effet cytocide significatif du PEG par rapport à la solution de contrôle (Crowson et al., 1987, Surgical Res. Com. 2 : 97-101).

Les Inventeurs ont maintenant mis en évidence la capacité inattendue du PEG et de ses dérivés, à prévenir ou guérir les cancers colorectaux. Dans le cadre de son utilisation comme adjuvant, il est connu d'utiliser le PEG comme véhicule de molécules diverses notamment de chimiothérapie anticancéreuse, comme par exemple la doxorubicine, mais le PEG n'a jamais été proposé jusqu'à ce jour pour prévenir ou guérir des cancers.

L'utilisation du PEG selon l'invention trouve tout particulièrement son application pour prévenir l'apparition de cancers colorectaux chez les sujets génétiquement atteints de polypose familiale (FAP) ou de syndrome de Lynch (HNPCC) et de façon générale, chez les personnes âgées de 65 ans ou plus ou présentant des facteurs de risques, comme par exemple des polypes intestinaux, et qui pourraient recevoir un traitement préventif chronique.

La présente invention envisage plus particulièrement l'utilisation des PEG et ses dérivés de poids moléculaire supérieur à environ 400 et de préférence supérieur à environ 1000, et tout préférentiellement de poids moléculaire élevé, de l'ordre d'environ 3000 à 9000 daltons.

Le polyéthylènepolypropylène glycol, aussi connu sous la dénomination de "pluronic F68" ou "poloxamer 188" et désigné ci-après "PLU", présente un poids moléculaire de 8350. Il est utilisé comme additif alimentaire pour ses propriétés surfactantes, lubrifiantes et anti-mousse. Le pluronic F68 répond à la formule (II) suivante :

HO (CH₂CH₂O)₇₅(CH(CH₃)CH₂OH)₃₀(CH₂CH₂O)₇₅H

Le PLU est commercialisé comme médicament laxatif sous le nom d'Idrocol®, qui est un laxatif osmotique agissant en augmentant la teneur en eau des fèces et favorisant leur glissement sur les parois intestinales (Vidal 1997, p. 814). Le PLU est aussi commercialisé comme médicament sous le nom d'Alkenide® pour des applications protectrices ou cicatrisantes, sous la forme d'une solution pour application locale et pour bain, notamment pour le nettoyage et le traitement d'appoint des affections de la peau et des muqueuses (Vidal, 1997, p. 48).

On entend par dérivé du PLU et du PEG, les composés de formules I ou II dont un ou plusieurs des groupes chimiques réactionnels sont modifiés ou substitués tout en conservant les capacités de prévention ou de guérison des cancers. Notamment, on entend par dérivé du PLU des polyéthylènepolypropylène glycols où l'enchaînement des blocs CH2CH2O et CH(CH3)CH2OH sont différents de ceux de la formule II.

L'invention vise en particulier l'utilisation des dérivés du PEG et du PLU présentant des propriétés d'hydrosolubilité et lubrifiante du niveau du PEG et du PLU en solution. L'homme du métier est à même de tester les capacités de prévention ou de guérison des cancers colorectaux de ces dérivés du PEG à partir des tests rapportés dans la partie expérimentale donnée plus loin.

Les travaux de recherches réalisés par les Inventeurs ont permis de mettre en évidence les propriétés remarquables de prévention et traitement des cancers colorectaux des laxatifs osmotiques non fermentés par rapport à d'autres types de laxatifs. Ainsi, parmi tous les laxatifs testés, le PEG et le PLU sont les seuls efficaces. Ainsi, les laxatifs testés n'appartenant pas à cette famille n'ont eu aucun effet préventif sur la cancérogenèse colique. Au contraire, la gomme karaya, qui est un laxatif de lest fermenté, semble plutôt promotrice. L'huile de paraffine, qui est un laxatif lubrifiant non fermenté, n'a aucun effet. Comme indiqué précédemment, les autres laxatifs fermentés, comme les fibres de lest (son de blé, psyllium, etc...) ou les sucres, qui sont des laxatifs osmotiques fermentés (lactulose, etc...) ont été largement étudiés et seraient légèrement protecteurs contre la cancérogenèse du colon mais sans jamais présenter les qualités des laxatifs osmotiques non fermentés mis en évidence dans le cadre de la présente invention.

L'invention concerne donc aussi l'utilisation d'un laxatif osmotique non fermenté en tant que principe actif pour la préparation d'une composition pharmaceutique caractérisée en ce qu'elle comprend comme principe actif du polyéthylène-glycol ou un dérivé de celui-ciau moins un laxatif osmotique non fermenté associé dans ladite composition avec un véhicule pharmaceutiquement acceptable, pour la prévention et le traitement des cancers colorectaux.

L'invention concerne tout particulièrement comme principe actif le PEG ou le PLU ou un dérivé de ceux-ci ou leur mélange.

Comme indiqué précédemment, l'invention se rapporte à l'utilisation d'un laxatif osmotique non fermenté en tant que principe actif pour la préparation de compositions comprenant comme principe actif du PEG et/ou un de ses dérivés de poids moléculaire supérieur à environ 400 et de préférence supérieur à environ 1000, et tout préférentiellement de poids moléculaire élevé, de l'ordre d'environ 3000 à 9000 daltons.

Le PEG n'étant vraisemblablement pas absorbé, l'invention se rapporte plus particulièrement à l'utilisation d'un laxatif osmotique non fermenté en tant que principe actif pour la préparation de toute composition pharmaceutique dans laquelle le PEG ou un dérivé de celui-ci est associé à un véhicule pharmaceutique sous une forme permettant l'administration per os ou en suppositoire ou encore en lavement ou sous toute autre forme permettant d'agir sur la paroi intestinale.

Les compositions sont dosées de façon à permettre une administration d'environ 0,2 à 0,8 g/jour de laxatif osmotique non fermenté par kilogramme de poids d'homme ou d'animal à traiter.

Ainsi, les compositions pharmaceutiques sont dosées de façon à permettre une administration de 10 à 80 g de laxatif osmotique non fermenté par jour. Ces valeurs ont été déterminées à partir des résultats expérimentaux obtenus chez le rat, sur la base des rapports de surface corporelle (équivalente au poids métaboliques, PO.72) Ainsi une administration quotidienne de 80 g de PEG est celle envisagée pour un individu d'environ 100 kg. L'extrapolation du rat à l'homme peut aussi se faire sur la base du poids sec d'aliment consommé par jour. Ce mode de calcul conduit à une dose efficace de laxatif osmotique non fermenté de 20 g par jour pour un individu moyen.

Les dosages ci-dessus concernent avantageusement l'administration orale du PEG et plus particulièrement d'un PEG 8000.

Tous véhicules pharmaceutiquement acceptables et compatibles avec les modes d'administration ci-dessus peuvent être utilisés dans les compositions pharmaceutiques.

D'autres avantages et caractéristiques de l'invention apparaîtront des exemples qui suivent concernant la mise en évidence des propriétés anti-cancéreuses du PEG et du PLU chez le rat.

### I - Exemple 1 : Expérience avec le PEG.

### 1) Matériel et méthodes.

L'expérience qui a permis de montrer l'effet anti-cancéreux du PEG chez le rat comportait 8 groupes de 10 à 20 rats initiés par l'azoxymethane (AOM). En effet afin d'étudier l'effet de l'hydratation des selles sur la cancérogenèse, certains groupes ont reçu un régime salé (3 à 6 %, de NaCl), d'autre un régime liquide (conduisant a des consommations d'eau de 150 et 200 % des témoins), ou avec une restriction de l'apport d'eau (90 % du témoin), un autre enfin traité par un diurétique (le furosemide). Les résultats présentés ci-après ne concernent que le groupe témoin et le groupe traité au PEG.

Des rats Fischer 344, femelles âgées de 4 semaines (Iffa Credo, L'Arbresles 69), ont été gardés 5 jours à leur arrivée, puis ils ont reçu une injection intrapéritonéale d'azoxymethane (20 mg/kg poids vif, dans NaCl 9 g/l, Sigma, St Quentin 38). Les rats ont été repartis, 7 jours plus tard, au hasard par 2 rats par cage du type cage suspendue métallique en grille inox, dans une animalerie climatisée (22°C ± 2°C) avec un cycle de jour et nuit de 12H. Les rats ont reçu ensuite ad libitum, l'un des régimes prévus au protocole, dans des ramequins hémisphériques en porcelaine renouvelés toutes les semaines. L'eau a été donnée dans des biberons en plastique à raison d'un biberon par cage.

Vingt rats témoins ont reçu un régime semi-purifié en poudre de composition standardisée (20 % protéines et 5 % lipides, UAR, Villemoisson 91) (American Institute of Nutrition, 1977, Report of the American Institute of Nutrition Ad Hoc Committee on standards for nutritional studies, J. Nutr. 107: 1340-1345), et dix rats traités ont reçu le même régime auquel était ajouté du PEG 8000 (réf. 194839 [25322-68-31_ de chez ICN, Orsay 91). La quantité de PEG a été ajustée chaque semaine en fonction du poids des rats de façon à leur apporter 3g/kg/j (environ 5% du poids sec de l'aliment). Les régimes ont été donnés une semaine après l'injection du cancérigène, et pendant 100 jours, jusqu'au sacrifice des animaux.

Les rats ont été pesés avant l'injection, et toutes les semaines jusqu'au sacrifice. L'apport énergétique et la consommation quotidienne d'eau et d'aliment de chaque rat ont également été enregistrés 1 jour par semaine (le mardi). Après 100 jours de régime expérimental, les rats de chaque groupe ont été tués par asphyxie au CO₂, ouverts, et leur côlon a été enlevé, vidé par injection de tampon de Krebs' Ringer, ouvert et fixé à plat entre deux feuilles de papier-filtre (codées) dans du formol tamponné (10 %). Après au moins 12h, les côlons ont été colorés au bleu de méthylène (15 minutes, 0,2 % dans Krebs' Ringer). Les microadénomes (*aberrant crypt foci,* ACF) ont été comptés (nombre et taille) au microscope (x40) par un seul observateur pour l'ensemble de l'expérience, en double aveugle (Bird, R.P, 1987, Cancer Lett. 37 : 147-151)( Bruce, W.R., Archer, M.C., Corpet. D.E.. Medline, A., Minkin, S., Stamp, D., Yin, Y. and Zhang, X.M., 1993, Mutation Research. 290 : 111-118).

Les mesures complémentaires suivantes ont également été effectuées :
- Vitesse de transit : le temps de transit d'un marqueur de la phase solide du contenu digestif, le chrome, a été mesuré.
- Teneur en eau des contenus : les fèces ont été pesées et comptées 1 jour/semaine (le mercredi, puis le poids frais et sec de ces fèces et donc le taux d'humidité ont été déterminés
- Dosage de composés fécaux : acides biliaires, cytotoxicité de l'eau fécale. Ces dosages ont été réalisés pour tester l'hypothèse que la protection contre le cancer du côlon vient de la dilution des composés promoteurs ou carcinogène dans l'eau fécale.

Trois études complémentaires ont été réalisées. La première, H2, a pour but d'étudier la relation entre la dose de PEG et l'effet protecteur. La seconde, H4, est destinée à montrer l'effet du PEG à court terme.La troisième, H3, est destiné à montrer l'effet du PEG sur de véritables cancers de l'intestin chez le rat.

La relation entre la dose de PEG et l'effet protecteur a été étudiée chez 42 rats Fischer 344, mâles, de 4 semaines, fournis par Iffa-Credo (Lyon). Une semaine après leur arrivée les rats reçoivent une injection unique d'azoxyméthane (20 mg/kg). On les a répartis au hasard en 4 lots, et nourris avec une alimentation standard AIN76 en poudre contenant 0 ; 0,5 ; 2 ou 5 % de PEG. Après 5 semaines de régime expérimental, les rats ont été sacrifiés, les côlons préparés comme expliqué précédemment.

L'effet du PEG à court terme a été étudié chez 40 rats Fischer 344 femelles, répartis au hasard en 4 lots. Dès son arrivée, et pendant 16 jours, l'un des lot de 10 rats a reçu un aliment AIN75 contenant 5% de PEG (lot I). Une semaine après leur arrivée, tous les rats ont reçu une injection unique d'azoxyméthane (20 mg/kg). Un lot témoin a reçu un régime standard AIN76 en poudre, et de l'eau de boisson pure, *ad libitum* (lot T). Une semaine après l'injection d'azoxyméthane, deux lots expérimentaux ont reçu du PEG à 5%, soit dans l'aliment (lot A), soit dans l'eau de boisson (lot E), pendant 30 jours. Après 30 jours de régime expérimental, les rats ont été sacrifiés, les côlons préparés comme expliqué précédemment.

Une troisième étude complémentaire, H3, a été réalisée pour mesurer l'effet du PEG sur l'apparition de tumeurs macroscopiques et de cancers du côlon chez le rat. Trente rats males F344 ont reçu 7 jours après une injection du cancérigène azoxyméthane (20 mg/kg) un aliment contenant 20% de caséine cuite (2h à 180°C) et 20% de saindoux. Cet aliment est fortement promoteur de la cancérogènèse chez le rat (Corpet et al., Cancer Research, 1990, 50:6955). Dix rat choisis au hasard parmi les 30 ont reçu en plus 5% de PEG dans cet aliment. Les rats ont été euthanasiés 88 jours après après l'injection du cancérigène et l'on a compté les tumeurs visibles sur chaque côlon et celles dont la surface était supérieure à 1 mm² ont été prélevées pour une analyse histologique classique après coupe mince (5 microns) et coloration Hemalun-Eosine.

### 2) Résultats.

Les résultats présentés dans les tableaux 1, 2 et 3 ci-dessous montrent une inhibition extrêmement importante de la cancérogenèse colique par le PEG, quel que soit le critère retenu.

**Tableau 1:**

| Nombre de microadénomes par côlon. | | |
|---|---|---|
| Groupe | Nombre de rats | Nombre ACF/rat |
| Témoins | 20 | 107 ±_ 43 |
| PEG | 10 | 6 _± 6 |
| p | | < 0,0001 (test de Welch, variances inégales) |

**Tableau 2 :**

| Multiplicité ou nombre de cryptes par microadénome. | | |
|---|---|---|
| Groupe | Nombre de rats | Nombre cryptes/ACF |
| Témoins | 20 | 2,9 ± 0,4 |
| PEG | 8 | 1,3 _± 0,4 |
| p | | < 0,0001 (test Student) |

**Tableau 3 :**

| Incidence des gros microadénomes de 3 cryptes et plus et nombre moyen de gros microadénomes par rat. | | | |
|---|---|---|---|
| Groupe | Nombre de rats | Incidence | Nombre de gros ACF |
| Témoins | 20 | 20 (100 %) | 58 ± 26 |
| PEG | 10 | 4 (40 %) | 0,7 ± 1,2 |
| p | | 0,0004 (test exact de Fisher) | 0,00001 (test de Mann-Whitney) |

Il convient de noter que deux rats ne présentaient pas de microadénome. Aussi, le tableau 2 ne prend en compte que les 8 rats ayant au moins un microadénome.

Les résultats non rapportés montrent que les rats traités au PEG ont eu une croissance semblable aux témoins, ont consommé autant d'aliments, et ont bu autant d'eau que les témoins. Leur excrétion fécale est par contre beaucoup plus importante :
- Témoins: 1,5 ± 0,08.
- Traités PEG: 3,46 ±_ 0,39 g/j,
- P < 0,0001.

De plus, le PEG a beaucoup augmenté le poids du cæcum (témoins 2,1 ± 0,4 contre traités 6,6 ± 0,9), et la teneur en eau des fèces (témoins 12,6 ± 2,4 contre traités 38,6 ± 5,0 et p<0,001.

Les trois études complémentaires H2, H3 et H4, dont les méthodes sont décrites plus haut, ont largement confirmé l'effet protecteur du PEG contre la cancérogenèse colique chez le rat.

L'étude de la relation entre la dose de PEG et l'effet protecteur a permis d'observer une relation très nette entre la dose de PEG administrée et l'effet protecteur.

Chez les groupes de rats mâles ayant reçu 0 ; 0,5 ; 2 ou 5% de PEG dans l'aliment pendant 5 semaines, on a détecté respectivement 127 ± 52, 110 ± 45, 88 ± 20 et 16 ± 10 microadénomes par côlon (moyenne de 14, 8, 10 et 10 rats, ± écart type). L'effet global est très significatif (ANOVA p<0,0001), mais seules les doses 2 et 5% de PEG ont diminué significativement le nombre d'ACF. La multiplicité des ACF (en gros, leur taille; précisément le nombre de crypte aberrante par ACF) était respectivement de 2,3 ; 2,2 ; 2,1 et 1,8 (écart type poolé de 0,2), l'effet global étant très significatif (p=0,0004), mais seule la dose de 5% ayant un effet significatif comparé au témoin.

On peut voir pour ces deux paramètres de la cancérogénèse la relation entre la dose de PEG et l'effet protecteur est très net, même si la dose de 5% est seule vraiment efficace. Cette étude montre enfin que le PEG est efficace chez les mâles, comme chez les femelles.

L'effet du PEG à court terme montre que l'administration de 5% de PEG pendant 30 jours, via l'aliment ou l'eau de boisson, diminue fortement la cancérogenèse du côlon, mais moins que sur une durée plus longue, par exemple de 100 jours.

Par contre, le PEG n'a aucun effet protecteur lorsqu'il est administré pendant la période d'initiation des tumeurs. Le nombre d'ACF par côlon chez les animaux témoins (T), ou ayant reçu le PEG pendant la période d'initiation (16 jours, I), ou après l'initiation (30 jours) soit dans l'aliment (A), soit dans l'eau de boisson (E) était T: 65 ± 31, I: 63 ± 31, A: 8 ± 8, E: 9 ± 10 (moyenne de 10 rats ± écart type).

Ces résultats sont très significatifs (ANOVA p<0.0001), et les groupes A et E sont similaires, et très différents du groupe témoin T (p<0.01), lui même identique au groupe I. Les différences entre groupes et les niveaux de signification sont similaires pour la multiplicité des ACF: T: 2,0 ; I: 1,9 ; A: 1,6 ; E: 1,7, et pour le nombre de gros ACF (plus de 3 cryptes/ACF): T: 5,6 ; I: 3,9 ; A: 0,3 ; E: 0,4. Cette étude confirme l'effet protecteur du PEG sur la cancérogenèse colique induite chimiquement chez le rat.

Dans l'étude H3 de cancérogénèse accélérée, chez des rats ayant reçus une alimentation très grasse contenant de la caséine cuite, le PEG a très fortement réduit le nombre et l'incidence des tumeurs et des cancers. Au total, 41 tumeurs ont été détectées chez les 20 rats témoins, contre 1 seule chez les 10 qui recevaient du PEG. L'incidence des tumeurs étaient de 14/20 chez les témoins contre 1/10 pour les rats "PEG" (p = 0,005, test exact de Fischer). Le nombre de tumeurs par rat était de 2,1 chez les témoins contre 0,1 chez les rats PEG (p = 0,003, test de Mann-Withney). Les résultats des analyses histologiques, vérifiés par anatomo-pathologiste, montrent que les rats témoins avaient au moins 15 adénocarcinomes (cancers) dont 6 invasifs, et 12 adénomes (polype bénin). La seule tumeur détectée chez les rats recevant du PEG était un adénome. Cette expérience H3 permet d'affirmer que le PEG protège les rats contre le cancer du côlon induit chimiquement.

Au total, quatre études indépendantes, réalisées dans des conditions variées, par plusieurs expérimentateurs (en particulier pour la lecture des microadénomes), montrent l'effet protecteur important du PEG sur la cancérogenèse colique. L'observation de cet effet protecteur ne peut pas être due au hasard.

Dans la discussion qui suit, le pouvoir protecteur du PEG est comparé à celui des autres molécules censées permettre une "chimioprévention" de la cancérogenèse colorectale. Le PEG est, et de très loin, le produit le plus efficace.

### 3) Discussion.

*Le tableau 4 ci-dessous présente l'efficacité de différents agents sur la cancérogenèse colique chez le* rat. Ce tableau présente un échantillon de 20 agents, dont les 6 plus efficaces, parmi les 80 identifiés dans la littérature.

Chaque valeur est le rapport entre la valeur trouvée chez les témoins ayant reçu un cancérigène, et la valeur trouvée chez les animaux traités par un agent préventif avant ou après le cancérigène. Plus la valeur est forte, plus l'agent est efficace.

**Tableau 4**

| Agent | Réf | Nbre ACF/rat | Cryptes/ACF | Gros ACF |
|---|---|---|---|---|
| PEG | 1 | 18 | 2,28 | 104 |
| Perilla oil | 2 | 3,9 | 1,09 | |
| Inulin+bifid.longum | 7 | 3,7 | | 2,3 |
| Piroxicam | 8 | 2,8 | | 8 |
| DHA/PhiP | 9 | 2,8 | 1,13 | |
| Hesperidin 1000ppp | 11 | 2,6 | 1,25 | |
| Auraptene 500ppm | 11 | 2,3 | 1,33 | |
| 13cis retinoic | 12 | 2,0 | | 1,9 |
| Dehydroepiandr. | 4 | 1,9 | 1,06 | |
| oxothiazolidine | 4 | 1,9 | 1,09 | |
| Sulindac | 5 | 1,7 | 1,10 | |
| Aspirin | 12 | 1,7 | | 2,7 |
| Acetoxychavicol acet. | 11 | 1,7 | 1,13 | 2,1 |
| piroxicam | 5 | 1,6 | 1,10 | |
| Fish oil K85 withAOM | 3 | 1,6 | | 1,8 |
| DHA/AOM | 10 | 1,6 | 1,08 | 2,0 |
| N-acetylcysteine | 4 | 1,5 | 1,10 | |
| Inulin 10% | 6 | 1,5 | | 1,4 |
| Vit. D3 | 8 | 1,3 | | 1,8 |
| Fish oilK85 wk17-23 | 3 | 1,2 | 1,08 | 1,6 |

La première colonne (Nb ACF/rat) correspond a l'apparition de microadénomes, les deux autres colonnes (crypt/ACF et Gros ACF) sont des marqueurs de la croissance des microadénomes.

Les résultats rapportées dans le tableau 4 ont été établis à partir des données publiées indiquées en références 2 à 12 dans le tableau 5 ci-dessous.

**Tableau 5**

| Publication | ACF/rat | Crypt/ACF | Large ACF |
|---|---|---|---|
| - Contrôle | 107 ± 43 | 2,9 ± 0,4 | 58 ± 26 |
| - PEG | 6 ± 6 | 1,3 ± 0,4 | 0,7 ± 1,2 |
| *Onogi et al. Carcinogenesis, 1996, 17, 1291-1296.* | | | |
| - Olive oil | 155 | 1,68 | |
| - Perilla oil | 40 | 1,54 | |
| *Paulsen et al.Pharmacol. & Toxicol., 1998,* 82, 1291-1296. "Fish oil concentrate K85 enriched EPA & DHA 3g/kgBW wk0-6, 2xAOM wk0&1". | | | |
| - Contrôle wk6 | 217 | | 26 |
| - K85 3mg withAOM wk0-6 | 139 | | 14 |
| - K85 1,5mg postAOM wk2-6 | 181 | | 25 |
| - Contrôle wk23 | 96 | 5,2 | 11 |
| - K85 2,2mg postAOM wkl7-23 | 80 | 4,8 | 7 |
| *Pereira-Khoury, Cancer Lett., 1991, 61 :* 27-33. "Agent wk-1 to +4, 2x15mg AOM at wk 0". | | | |
| - Contrôle | 228 ± 32 | 2,59 ± 0,14 | |
| - N-acetylcystéine | 151 ± 21 | 2,35 ± 0,07 | |
| - Dehydroepiandrosterone | 121 ± 19 | 2,44 ± 0,07 | |
| - Oxothiazolidine | 121 ± 11 | 2,38 ± 0,08 | |
| *Pereira et al., Carcinogenesis, 1994, 15 : 1049-1054."Agent given for day 1-35, AOM on d. 7 & 14".* | | | |
| - Contrôle | 26 ± 2,2 | 2,04 ± 0,04 | |
| - Pyroxicam | 16 ± 2,8 | 1,84 ± 0,1 | |
| - Sulindac | 15 ± 3,2 | 1,82 ± 0,14 | |
| *Reddy et al., Carcinogensis, 1997, 18 : 1371-1374. "Inulin or oligofruct for 10 wks, AOM at wk 3 & 4".* | | | |
| - Contrôle | 120 | | 56 |
| - Inulin 10% | 78 | | 39 |
| *Rowland et al., Carcinogenesis, 1998, 19 : 281-285. " Bifid. & inulinx12 wks, 7 d postAOM".* | | | |
| - Contrôle | 130 | | 37 |
| - Inulin+Bifid.longum | 35 | | 16 |
| *Salim et al., Japanese Journal of Cancer Research, 88 : 1052-1062. " Vit. D3 postDMH for 16 wks".* | | | |
| - Contrôle | 352 | | 120 |
| - Vit. D3 10ppm | 269 | | 66 |
| *Takahashi et al., Carcinogenesis, 1997, 18, 1937-1941. "ACF induced by 10x (PhIP) & DHA 4 h before each dose".* | | | |
| - Contrôle | 22 | | 2,69 |
| - DHA | 8 | | 2,39 |
| *Takahashi et al., Carcinogenesis, 1997, 18, 1937-1941. (Correl. With adenocarcinoma) "ACF induced by 20x AOM & 1ml DHA with each dose".* | | | |
| - Contrôle 12w | 308 ± 64 | 3,2 | 114 |
| - DHA 12w | 191 ± 40 | 2,95 | 56 |
| *Tanaka et al., Carcinogenesis, 1997*,, *18 : 2155-2161. "(Correl.*/*carcinomia) ACF induced by 3x AOM 15mg*/*kg".* | | | |
| - Contrôle p.957 | 89 ± 17 | 2,77 | |
| - Hesperidin 1000 ppm | 34 ± 5 | 2,22 | |
| - Contrôle p.1113 | 118 ± 28 | 2,54 | 60 |
| - Acetoxychavicol acet.100 ppm | 70 ± 10 | 2,24 | 28 |
| - Contrôle p.2155 | 157 ± 21 | 2,91 | |
| - Auraptene | 69 ± 6 | 2,19 | |
| *Wargovich et al., Int. J. Cancer., 1995, 60 : 515-519. "AOM, then 4 wks, then agent given for 4 wks".* | | | |
| - Contrôle/asp | 175 | | 55 |
| - Aspirin 0,2g/kg | 100 | | 20 |
| - Contrôle /pir | 125 | | 40 |
| - Piroxicam 0,2 | 45 | | 5 |
| - Contrôle /ret | 140 | | 57 |
| - 13cis Retinoic | 70 | | 30 |

Les résultats rapportés dans le tableau 4 montrent que le PEG est l'agent le plus efficace dans la prévention de la cancérogenèse côlorectale induite chimiquement chez le rat. Le tableau 4 permet de comparer l'efficacité du PEG avec celle d'autres agents. De plus, la plupart des agents présentés dans le tableau 4 ont été administrés avant et pendant l'initiation des tumeurs. Ils ont donc pu agir tout simplement en bloquant le cancérigène ou en inhibant son métabolisme.

Comme il est très peu probable que les cancers côlorectaux de l'homme soient initiés par l'azoxyméthane, cela limite fortement l'intérêt de ces agents. Le PEG a par contre été administré une semaine après l'injection du cancérigène azoxymethane.

Le PEG a donc eu un effet curatif sur les microadénomes et les cancers, indépendant du cancérigène qui est métabolisé et excrété en moins de 24 heures.

### 4) Effet de PEGs de différent poids moléculaire.

### - Méthode.

Un groupe de 32 rats F344 a reçu une injection du cancérigène azoxyméthane (20 mg/kg), et 7 jours plus tard a été réparti en un groupe témoin de 12 rats et 5 groupes expérimentaux de 4 rats. Les groupes expérimentaux ont reçu dans l'eau de boisson 5% de PEG de poids moléculaire compris entre 400 et 20 000. Le PEG 8000 a été donné à deux groupes à des doses de 5 ou 10%.

### - Résultats.

Les résultats sont donnés dans le tableau 6 ci-dessous. Il apparaît que le PEG 8000 à la dose de 5% a été plus efficace pour supprimer les ACF. Le tableau 6 montre que les nombre d'ACF par rat, ou le nombre de gros ACF par rat, suit une courbe en U en fonction du poids moléculaire du PEG ingéré. Le point bas de cette courbe en U correspond au groupe qui a reçu 5% de PEG 8000. Cependant la différence entre les PEGs 3350 et 8000 n'est pas significative du fait sans doute du faible nombre de rats par groupe.

Comme dans l'expérience rapportée à l'exemple 2 ci-après, il n'y pas de corrélation entre l'effet laxatif des produits, mesurés par le pourcentage d'eau dans les selles, ou le poids des selles excrétées en 24 heures, ou le poids de caecum) et les données de cancérogénèse (nombre d'ACF, taille des ACF, nombre de gros ACF).

**Tableau 6**

| PEG | | Nombre d'ACF | Gros ACF 4 et plus | % eau dans les selles | | Poids fécès |
|---|---|---|---|---|---|---|
| P.M. | Dose | | | De 24 h | Fraîches | |
| Témoin | 0% | 135 | 18,7 | 11,8 | 28 | 1,4 |
| 400 | 5% | 97,5 | 5,2* | 48,3* | 74* | 2,8* |
| 3350 | 5% | 46,5* | 5,7* | 31,7* | 66* | 2,6* |
| 8000 | 5% | 23,3* | 1,2* | 35,6* | 67* | 3,0* |
| 8000 | 10% | 43,3* | 3,0* | 47,0* | 66* | 4,5* |
| 20000 | 5% | 64,8* | 6,2* | 29,7* | 67* | 4,2* |

| | | | | | | |
|---|---|---|---|---|---|---|
| * : différence avec le témoin très significative, p < 0,01. | | | | | | |

### II - Exemple 2 : Expériences avec le PLU et le PEG.

### 1) Matériel et méthodes.

L'expérience a consisté à donner à 44 rats F344 mâles âgés de 5 semaines une injection de cancérigène azoxyméthane (20 mg/kg), puis à les répartir 7 jours plus tard en différents lots recevant une alimentation en poudre, basée sur la formule de l'AIN 76, contenant 5% de PEG ou de PLU ou d'autres substances indiquées dans le tableau 7 ci-dessous.

**Tableau 7**

| ESSAI | COMPOSITION | RATS |
|---|---|---|
| Témoin | AIN 76 poudre | 12 |
| PLU | AIN 76 poudre à 5% Pluronic F 68 | 4 |
| PEG | AIN 76 poudre à 5% PEG 8000 ICN | 4 |
| CAR | AIN 76 poudre à 5% carboxymethylcellulose | 4 |
| PVP | AIN 76 poudre à 5% polivinylpyrrolidone | 4 |
| PFA | AIN 76 poudre à 5% PEG fatty alcohol ether | 4 |
| POE | AIN 76 poudre à 5% polyoxyéthylène fatty acids esters | 4 |
| KAR | AIN 76 poudre à 5% gomme Karaya | 4 |
| HUI | AIN 76 poudre à 5% huile de paraffine | 4 |

### 2) Résultats et discussion.

Les résultats et la discussion ci-dessous ne concernent que le PEG et le PLU car les autres produits testés n'ont présenté aucun effet protecteur ou, au contraire, un effet promoteur de la croissance des microadénomes (ACF).

On observe comme à l'exemple 1 l'effet très protecteur du PEG : tous p<0,001, avec moins d'ACF : 3,72 contre 52,8 chez 11 témoins, et moins de gros ACF : 2,5 contre 39 (2 et plus), 1,25 contre 17 (3 et plus), 0,5 contre 6,8 (4 et plus).

En ce qui concerne le PLU, on observe un effet plus protecteur qu'avec le PEG : tous p<0,001, moins d'ACF que les témoins : 0,75 contre 52,8, et beaucoup moins de gros ACF : 0,25 contre 39 (2 et plus), 0,25 contre 17 (3 et plus), 0,0 contre 6,8 (4 et plus).

Malgré le faible nombre de rat, il semble que le PLU soit plus efficace que le PEG. Toutefois, il est difficile de comparer les résultats obtenus avec 4 rats PLU et 4 rats PEG, car les différences ne sont pas suffisament significatives :
Nombre d'ACF : 10,75 contre 3,75
Nombre de gros ACF : 2 et plus : 0,25 contre 2,5 p=0,23 ; 4 et plus : 0,0 contre 0,5
Multiplicité : 1,5 contre 2,22, mais il n'y a que deux rat avec ACF par groupe.

En outre, le PLU pourrait présenter l'avantage d'être moins laxatif que le PEG chez l'homme. En effet, chez le rat, le PEG augmente le poids du cecum de 120% tandis que le PLU ne l'augmente que de 40% (TEM 3,3g, PEG 7,3g, PLU 4,8g, tous p<0,001). Par contre l'excrétion fécale est très augmentée par rapport aux témoins et similaire dans les groupes PEG et PLU (TEM 1,9g/j, PEG 4,8g, PLU 5,6g), de même que la teneur en eau des selles (TEM 30%, PEG 68%, PLU 64%). Enfin ni le PLU ni le PEG n'ont changé la prise de poids des rats traités par rapport aux témoins (TEM 269,9g, PEG 270,1g, PLU 267,7g).

## Revendications

1. Utilisation d'un laxatif osmotique non fermenté en tant qu'agent actif pour la préparation d'un médicament destiné au traitement et/ou à la prévention des cancers du côlon et/ou du rectum.

2. Utilisation selon la revendication 1, **caractérisée en ce que** le laxatif osmotique non fermenté est un polyol.

3. Utilisation selon l'une quelconque des revendications 1 ou 2, caractérisée en ce le laxatif osmotique non fermenté est le polyéthylène-glycol ou le polyéthylènepolypropylène glycol, un mélange ou un dérivé de ceux-ci.

4. Utilisation selon la revendication 3, **caractérisée en ce que** le polyéthylène-glycol ou le dérivé de celui-ci est de poids moléculaire supérieur à environ 400 et de préférence supérieur à environ 1000, et tout préférentiellement de poids moléculaire élevé, de l'ordre d'environ 3000 à 9000 daltons et avantageusement de l'ordre de 8000 daltons.

5. Utilisation selon l'une des revendications 1 à 4 pour la préparation d'un médicament destiné au traitement et/ou à la prévention des cancers du côlon et/ou du rectum chez les sujets génétiquement atteints de polypose familiale ou de syndrome de Lynch, ou encore chez les personnes âgées ou présentant des facteurs de risque.

## Patentansprüche

1. Verwendung eines nicht fermentierten osmotischen Laxativums als Aktivstoff für die Zubereitung eines Medikaments, das zur Behandlung und/oder Vorbeugung von Krebserkrankungen des Colons und/oder des Rektums bestimmt ist.

2. Verwendung nach Anspruch 1, **dadurch gekennzeichnet, dass** das nicht fermentierte osmotische Laxativum ein Polyol ist.

3. Verwendung nach einem der Ansprüche 1 oder 2, **gekennzeichnet dadurch, dass** das nicht fermentierte osmotische Laxativum Polyethylenglycol oder Polyethylenpolypropylenglycol, eine Mischung oder ein Derivat dieser ist.

4. Verwendung nach Anspruch 3, **dadurch gekennzeichnet, dass** das Polyethylenglycol bzw. sein Derivat ein Molekulargewicht höher als ca. 400 und vorzugsweise höher als ca. 1000 hat, und insbesondere vorzugsweise ein hohes Molekulargewicht in der Größenordnung von ca. 3000 bis 9000 Dalton und vorteilhafterweise in der Größenordnung von 8000 Dalton.

5. Verwendung nach einem der Ansprüche 1 bis 4 für die Zubereitung eines Medikaments, das zur Behandlung und/oder Vorbeugung von Krebserkrankungen des Colons und/oder des Rektums bei Personen bestimmt ist, die genetisch von einer familiären Polypose bzw. dem Lynch-Syndrom betroffen sind bzw. bei Älteren oder Personen, die Risikofaktoren aufweisen.

## Claims

1. Application of an unfermented osmotic laxative as an active agent for the preparation of a drug intended to treat and/or prevent cancer of the colon and/or rectum.

2. Application according to claim 1, **characterized in that** the unfermented osmotic laxative is a polycl.

3. Application according to one of the claims 1 or 2, **characterized in that** the unfermented osmotic laxative is the polyethylene-glycol or the polyethylene-polypropylene glycol, a mixture or a derivative of these.

4. Application according to claim 3, **characterized in that** the polyethylene-glycol or the derivative of the latter has a high molecular weight of approximately 400 and preferably greater than approximately 1000, with everything preferably having a higher molecular weight, of the order of approximately 3,000 to 9,000 daltons and advantageously of the order of 8,000 daltons.

5. Application according to one of the claims 1 to 4 for the preparation of a drug intended to treat and/or prevent cancer of the colon and/or of the rectum in the subjects who were genetically afflicted with familial polyposis or hereditary non-polyposis colon cancer or, furthermore, among elderly people or those exhibiting risk factors.
